# EUROPEAN PATENT APPLICATION

(11) **EP 4 752 901 A1**
(43) Date of publication of application: **03.06.2026**
(21) Application number: 25215606.2
(22) Date of filing: 13.11.2025
(51) Int. Cl.: G16H 30/40, G16H 40/63, G16H 50/20, G01N 1/00

(54) **DISPLAY SYSTEM, DISPLAY METHOD, AND DISPLAY PROGRAM FOR ASSESSING SAMPLE ANALYSIS DATA**

(30) Priority: 02.12.2024 JP 2024209484
(71) Applicant: Toyota Jidosha Kabushiki Kaisha, Toyota-shi, Aichi-ken 471-8571 (JP)
(72) Inventor: SAEKI, Kazuho, Toyota-shi, 471-8571 (JP)
(74) Representative: J A Kemp LLP

(57) **Abstract**

A display system, a display method, and a display program capable of assisting analysis that utilizes knowledge possessed by an experimenter are provided. A display system (1) according to the present disclosure includes an analysis subject data acquisition unit (111), a text acquisition unit (112), an extraction unit (113), and a display control unit (114). The analysis subject data acquisition unit (111) acquires analysis subject data including at least one of measurement data obtained by measuring samples and numerical data about the samples. The text acquisition unit (112) acquires text data about the samples. The extraction unit (113) extracts extraction information about the samples from the text data. The display control unit (114) displays at least one of the analysis subject data and a result of analysis of the analysis subject data in a differentiated manner in accordance with a difference between pieces of the extraction information.

## Description

### BACKGROUND

The present disclosure relates to a display system, a display method, and a display program.

Patent Literature 1 discloses a data analysis system that analyzes measurement data obtained by analyzing materials. The data analysis system disclosed in Patent Literature 1 receives measurement data through a communication apparatus, processes the measurement data using a trained machine learning model, and outputs a result of the analysis.

[Patent Literature 1] Japanese Unexamined Patent Application Publication No. 2021-092467

### SUMMARY

It should be noted that experimenters and developers obtain various kinds of knowledge in experiments. Such knowledge may include important information in analysis.

However, it is difficult to incorporate the above knowledge into analysis. Therefore, in the analysis system according to the related art, the above knowledge cannot be sufficiently utilized.

That is, in the analysis system according to the related art, there is a problem that knowledge obtained by an experimenter in an experiment cannot be sufficiently utilized. Patent Literature 1 does not disclose any technology that can solve such a problem.

The present disclosure has been made to solve such a problem, and an object thereof is to provide a display system, a display method, and a display program that are capable of assisting analysis that utilizes knowledge possessed by an experimenter.

A display system according to the present disclosure includes an analysis subject data acquisition unit, a text acquisition unit, an extraction unit, and a display control unit. The analysis subject data acquisition unit acquires analysis subject data including at least one of measurement data obtained by measuring samples and numerical data about the samples. The text acquisition unit acquires text data about the samples. The extraction unit extracts extraction information about the samples from the text data. The display control unit displays at least one of the analysis subject data and a result of analysis of the analysis subject data in a differentiated manner in accordance with a difference between pieces of the extraction information.

According to the above configuration, a user can intuitively know how the extracted difference between samples is reflected in analysis subject data and a result of analysis of the analysis subject data. As a result, a display system 1 according to the embodiment can assist analysis that utilizes knowledge possessed by an experimenter.

In the display system according to the present disclosure, the extraction information may include at least one of information about experimental conditions of the samples and information about physical properties of the samples.

In the display system according to the present disclosure, the display control unit may group the samples into a plurality of groups in accordance with the difference between pieces of the extraction information, and display at least one of the analysis subject data and the result of analysis of the analysis subject data in different forms for each of the groups.

In the display system according to the present disclosure, the analysis subject data may include the measurement data. Further, the display control unit may display the measurement data in different colors for each of the groups.

In the display system according to the present disclosure, the display control unit may display at least one of the analysis subject data and the result of analysis of the analysis subject data in different display areas for each of the groups.

The display system according to the present disclosure may further include an analysis unit configured to plot plot points corresponding to the samples on mapping data based on the analysis subject data. Further, the display control unit may display the plot points in different forms for each of the groups.

A display method according to the present disclosure performs the following steps.

Analysis subject data including at least one of measurement data obtained by measuring samples and numerical data about the samples is acquired.

Text data about the samples is acquired.

Extraction information about the samples is extracted from the text data.

At least one of the analysis subject data and a result of analysis of the analysis subject data is displayed in a differentiated manner in accordance with a difference between pieces of the extraction information.

A display program according to the present disclosure causes a computer to perform the following operations.

Analysis subject data including at least one of measurement data obtained by measuring samples and numerical data about the samples is acquired.

Text data about the samples is acquired.

Extraction information about the samples is extracted from the text data.

At least one of the analysis subject data and a result of analysis of the analysis subject data is displayed in a differentiated manner in accordance with a difference between pieces of the extraction information.

By the present disclosure, it is possible to provide a display system, a display method, and a display program that are capable of assisting analysis that utilizes knowledge possessed by an experimenter.

The above and other objects, features and advantages of the present disclosure will become more fully understood from the detailed description given hereinbelow and the accompanying drawings.

### BRIEF DESCRIPTION OF DRAWINGS

Fig. 1 is a block diagram showing a configuration of a display system according to a first embodiment;
Fig. 2 is a block diagram showing a configuration of a server according to the first embodiment;
Fig. 3 is a block diagram showing the configuration of the server according to the first embodiment;
Fig. 4 is a schematic screen diagram for explaining the configuration of the server according to the first embodiment;
Fig. 5 is a schematic screen diagram for explaining the configuration of the server according to the first embodiment;
Fig. 6 is a flowchart showing operations performed by the display system according to the first embodiment;
Fig. 7 is a block diagram showing a configuration of a server according to a second embodiment; and
Fig. 8 is a schematic screen diagram for explaining the configuration of the server according to the second embodiment.

### DESCRIPTION OF EMBODIMENTS

### <First Embodiment>

### (Configuration of Display system)

A first embodiment according to the present disclosure will be described hereinafter in detail with reference to the drawings. Firstly, a configuration of a display system according to this embodiment will be described in detail.

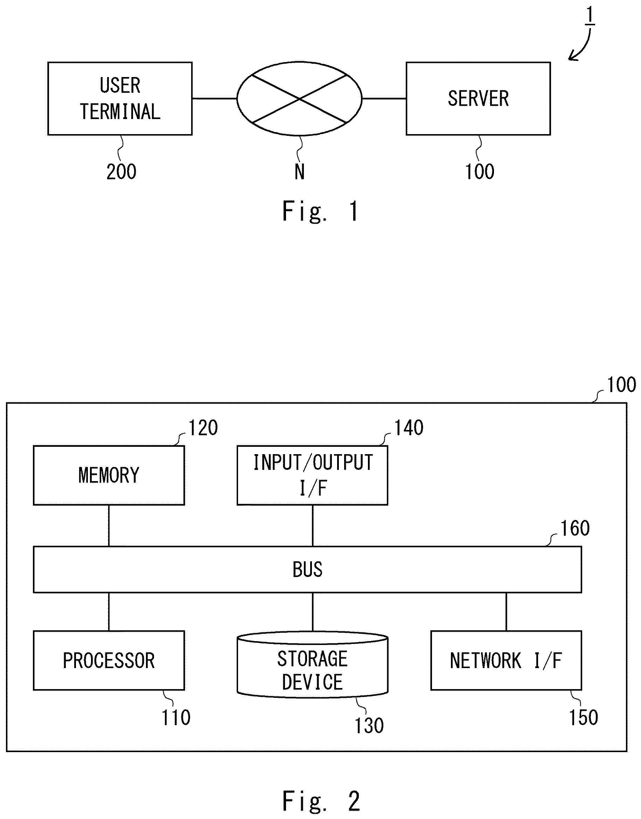

Fig. 1 is a block diagram showing a configuration of a display system according to the first embodiment. As shown in Fig. 1, a server 100 and a user terminal 200 are connected to a display system 1 according to this embodiment through a network N such as the Internet.

The display system 1 according to this embodiment is typically provided as a part of a data cloud type service used in material development or research and development, and is used as a system for promoting research and development using so-called Materials Informatics (MI) or data science.

The display system 1 stores various types of data about experimental samples. Further, the display system 1 analyzes the stored data based on an instruction from a user.

Note that various types of data about experimental samples described herein include analysis subject data (i.e., data to be analyzed) and text data described later.

In the display system 1, the user terminal 200 transmits the various types of data about the experimental samples to the server 100, and the server 100 analyzes the received data. Then the server 100 transmits results of the analysis to the user terminal 200, and the user terminal 200 displays the received results of the analysis.

The user terminal 200 according to this embodiment is a terminal operated by a user, and is typically a computer apparatus including a display apparatus.

The user terminal 200 transmits the various types of data about the experimental samples to the server 100 through the network N. Then the user terminal 200 receives the results of the analysis of the various types of data about the experimental samples from the server 100 through the network N.

The server 100 according to this embodiment receives the various types of data about the experimental samples from the user terminal 200 through the network N, and analyzes the received data. Then the server 100 transmits results of the analysis to the user terminal 200 through the network N.

Fig. 2 is a block diagram showing a hardware configuration of the server according to the first embodiment.

As shown in Fig. 2, the server 100 includes a processor 110, a memory 120, a storage device 130, an input/output interface 140, a network interface 150, and an internal bus 160.

The internal bus 160 is a data transmission path through which the processor 110, the memory 120, the storage device 130, the input/output interface 140, and the network interface 150 transmit and receive data to and from each other. However, the method for connecting the processor 110 and the like to each other is not limited to the bus connection.

The memory 120 is a main storage device implemented by using a Random Access Memory (RAM) or the like. Further, the storage device 130 is an auxiliary storage device implemented by using a hard disk, a Solid State Drive (SSD), a memory card, a Read Only Memory (ROM), or the like. The storage device 130 stores a program(s) for implementing desired functions.

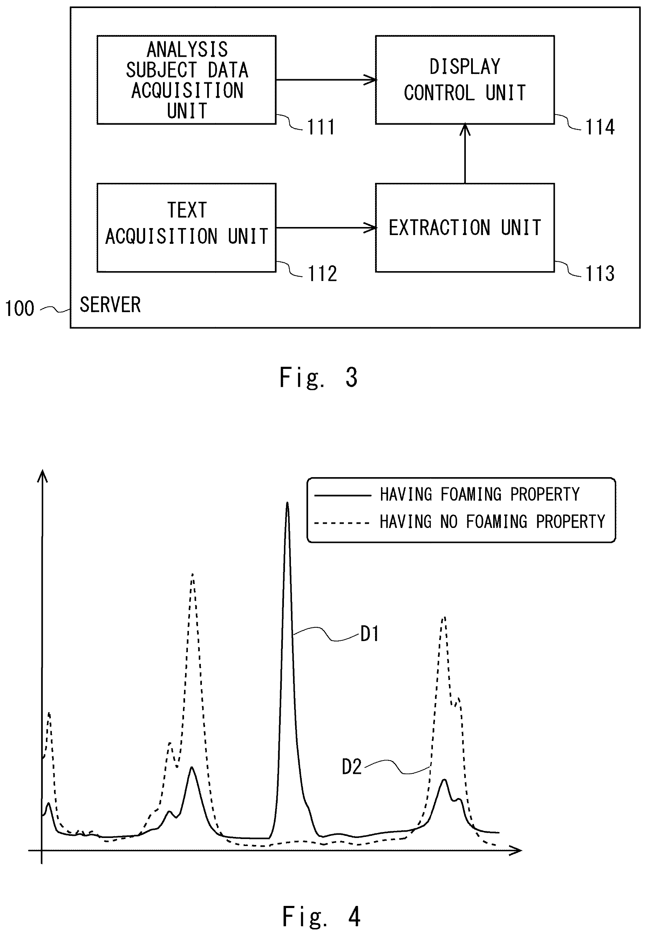

The processor 110 may be a Central Processing Unit (CPU), a Graphics Processing Unit (GPU), or a field-programmable gate array (FPGA). The processor 110 performs the functions of the respective functional blocks shown in Fig. 3 described later by loading the program(s) stored in the storage device 130 into the memory 120 and executing the loaded program(s).

The input/output interface 140 is an interface for connecting the server 100 to input/output devices. For example, an input device such as a keyboard and/or an output device such as a display apparatus may be connected to the input/output interface 140.

The network interface 150 is an interface for connecting the server 100 to the network.

The program can be stored and provided to a computer using any type of non-transitory computer readable media. Non-transitory computer readable media include any type of tangible storage media. Examples of non-transitory computer readable media include magnetic storage media (such as floppy disks, magnetic tapes, hard disk drives, etc.), optical magnetic storage media (e.g. magneto-optical disks), CD-ROM (compact disc read only memory), CD-R (compact disc recordable), CD-R/W (compact disc rewritable), and semiconductor memories (such as mask ROM, PROM (programmable ROM), EPROM (erasable PROM), flash ROM, RAM (random access memory), etc.). The program may be provided to a computer using any type of transitory computer readable media. Examples of transitory computer readable media include electric signals, optical signals, and electromagnetic waves. Transitory computer readable media can provide the program to a computer via a wired communication line (e.g. electric wires, and optical fibers) or a wireless communication line.

Fig. 3 is a block diagram showing a configuration of the server according to the first embodiment. As shown in Fig. 3, the server 100 according to this embodiment includes, as its functional blocks, an analysis subject data acquisition unit 111, a text acquisition unit 112, an extraction unit 113, and a display control unit 114.

The analysis subject data acquisition unit 111 acquires analysis subject data. More specifically, the analysis subject data acquisition unit 111 according to this embodiment acquires a plurality of pieces of analysis subject data from the user terminal 200 through the network N. The analysis subject data acquisition unit 111 outputs the acquired analysis subject data to the display control unit 114.

Further, the analysis subject data acquisition unit 111 may store the acquired analysis subject data in the storage device 130 each time it acquires analysis subject data. Further, when analysis described later is performed, the analysis subject data acquisition unit 111 may read the analysis subject data from the storage device 130 and output it to the display control unit 114.

Further, in this case, the analysis subject data acquisition unit 111 does not need to read all pieces of the analysis subject data stored in the storage device 130, and may instead read only the analysis subject data designated by a user and output it to the display control unit 114.

That is, the analysis subject data acquisition unit 111 may create a database in which the analysis subject data is stored. Further, the analysis subject data acquisition unit 111 according to this embodiment may be configured so that a user can select analysis subject data from the created database as appropriate.

Note that analysis subject data acquired by the analysis subject data acquisition unit 111 according to this embodiment is at least one of measurement data obtained by measuring samples and numerical data about the samples.

In other words, the analysis subject data acquisition unit 111 according to this embodiment acquires analysis subject data which is at least one of measurement data obtained by measuring samples and numerical data about the samples.

Note that measurement data obtained by measuring samples may be raw data output from a measuring device which has measured the samples, or data which has been subjected to analysis processing. Measurement data obtained by measuring samples may be any data recorded in computer-readable form.

Examples of measurement data obtained by measuring samples include spectrum data, waveform data, graph data, two-dimensional image data, and three-dimensional image data.

Examples of spectrum data include spectrum data measured by nuclear magnetic resonance spectroscopy (NMR), infrared spectroscopy (IR), ultravioletvisible spectroscopy (UV-vis), X-ray absorption spectroscopy (XAS), Raman spectroscopy, X-ray diffraction (XRD), small-angle X-ray scattering (SAXS), mass spectrometry (MS), or the like.

Further, examples of two-dimensional image data include image data captured by using an optical microscope, a scanning electron microscope (SEM), a transmission electron microscope (TEM), a computed tomography (CT), or the like.

Further, examples of three-dimensional image data include photographic data which is created by stacking tomograms taken by computed tomography (CT), model data created by Computer-Aided design (CAD), or the like.

Further, examples of waveform data include time-series data and displacement data. Examples of time-series data include acoustic data and vibration data, and any data whose numerical value changes with time can be used as analysis subject data. Examples of displacement data include a surface height and a surface profile of a sample, and any data whose numerical value changes as coordinates and other parameters change can be used.

Further, examples of other data include a cyclic voltammogram, a chart graph of gas chromatography (GC), and coordinate data such as a Crystallographic Information (CIF) file.

Numerical data about a sample may be, for example, numerical data defining an experimental condition of a sample or numerical data indicating the composition of a sample.

For example, when a sample is a composition, numerical data about the sample may be the content of each component contained in the composition. Further, when a sample is a product, numerical data about the sample may be, for example, numerical values related to reaction conditions such as a reaction temperature, a reaction time, a weight of a substrate used for reaction, and a reaction scale.

Further, analysis subject data may be data corresponding to both measurement data obtained by measuring samples and numerical data about the samples. Examples of data corresponding to both measurement data obtained by measuring samples and numerical data about the samples include physical property values of the samples.

Examples of physical property values of the samples include a mechanical property value typified by strength, hardness, toughness, abrasion resistance, etc., a physical property value typified by density, conductivity, a magnetic property, thermal conductivity, a thermal expansion rate, etc., and a chemical property value typified by corrosion resistance etc.

Further, analysis subject data is not limited to data related to only a sample, and may include, for example, a performance value of a product and a module created using a sample.

Examples of a performance value of a product and a module created using a sample may include a photoelectric conversion efficiency of a solar cell including a photoelectric conversion layer using a sample as a material and a numerical value obtained by evaluating the durability of a vehicle manufactured using a sample as a body material.

That is, analysis subject data acquired by the analysis subject data acquisition unit 111 may be data directly or indirectly defining the composition of a sample to be analyzed, or data directly or indirectly evaluating the performance of a sample to be analyzed.

Note that analysis subject data according to this embodiment is data in which information about experiment contents are recorded in a form other than text.

The text acquisition unit 112 acquires text data in which experiment contents of samples are recorded. The text acquisition unit 112 outputs the acquired text data to the extraction unit 113.

Further, the text acquisition unit 112 may store the acquired text data in the storage device 130 each time it acquires text data. Further, when analysis described later is performed, the text acquisition unit 112 may read the text data from the storage device 130 and output it to the extraction unit 113.

Further, in this case, the text acquisition unit 112 does not need to read all pieces of the text data stored in the storage device 130, and may instead read only the text data designated by a user and output it to the extraction unit 113.

That is, the text acquisition unit 112 may create a database in which text data is stored. Further, the text acquisition unit 112 according to this embodiment may be configured so that a user can select analysis subject data from the created database as appropriate.

Further, in the database described herein, text data may be associated with the above-described analysis subject data and stored. That is, the server 100 according to this embodiment may associate the acquired analysis subject data with text data for each sample and store them in the same database.

Note that, in this case, the text acquisition unit 112 may acquire the text data associated with the analysis subject data designated as analysis subject data by a user.

Note that the text data according to this embodiment is data of a document created by an experimenter who conducted an experiment on samples in order to record contents of the experiment, which data is recorded in the form of text.

For example, text data may be created by an experimenter inputting text to be recorded as experiment contents using input means such as a keyboard to the user terminal 200. In this case, the text acquisition unit 112 receives the input text data from the user terminal 200, thereby acquiring the text data in which the experiment contents of the samples are recorded. Note that, in this case, the text data may be referred to as an electronic experiment notebook.

Further, text to be recorded as experiment contents may be text recorded on a paper surface by an experimenter using a writing material etc. In this case, the text acquisition unit 112 may acquire image data of the paper surface in which the experiment contents are recorded by a user from the user terminal 200 and perform image processing on the acquired image data, thereby converting characters described in the paper surface into text data. Then the text acquisition unit 112 may acquire the converted text data as text data in which the experiment contents of the samples are recorded. Note that, in this case, text to be recorded as experiment contents may be a so-called experiment notebook.

As described above, the text acquisition unit 112 according to this embodiment may acquire text data originally recorded as electronic data, or text data generated by converting character information originally recorded on a paper surface.

That is, the text acquisition unit 112 may acquire text data by acquiring data other than text data and converting the acquired data, or by extracting it from the acquired data.

For example, the text acquisition unit 112 may extract text data from electronic data including text data and image data. Note that, an image described herein may be, for example, a photograph of the exterior of a sample, a sketch of an experimental instrument, an image showing spectrum data obtained by measuring a sample, or the like. That is, an image described herein may be any image to be described in an experiment notebook or an electronic experiment notebook.

The display system according to this embodiment displays at least one of analysis subject data and a result of analysis of the analysis subject data while differentiating them in accordance with a difference between pieces of information extracted from text data acquired by the text acquisition unit, the details of which will be described later.

That is, the display system according to this embodiment extracts a difference between samples from a document created by an experimenter to record experiment contents, and displays at least one of analysis subject data and a result of analysis of the analysis subject data while differentiating them in accordance with the extracted difference.

According to the above configuration, a user can intuitively know how the extracted difference between samples is reflected in analysis subject data and a result of analysis of the analysis subject data. As a result, the display system 1 according to this embodiment can assist analysis that utilizes knowledge possessed by an experimenter.

The extraction unit 113 acquires text data from the text acquisition unit 112. The extraction unit 113 extracts extraction information about experiment contents from the text data. The extraction unit 113 outputs the extracted extraction information to the display control unit 114.

Extraction information according to this embodiment is used to assist analysis of samples. Therefore, the extraction information according to this embodiment preferably includes, for example, at least one of information about experimental conditions of samples and information about physical properties of the samples.

The extraction information extracted by the extraction unit 113 is information obtained by extracting information about experiment contents included in text data in such a manner that it can be classified or evaluated. Therefore, the extraction information according to this embodiment may include, for example, numerical data in which a plurality of variables are recorded, or identification data in which identification information about various elements related to each sample is recorded.

In the above case, the extraction unit 113 may first extract an item related to at least one of information about experimental conditions of samples and information about physical properties of the samples.

Then the extraction unit 113 may extract numerical data as extraction data by converting the degree of the extracted item into numbers for each sample. Further, the extraction unit 113 may extract identification data as extraction data by extracting the identification information about the extracted item from text data.

In the above case, the extraction unit 113 may, for example, refer to a database in which words related to the experimental conditions and words related to the physical properties are recorded. Then the extraction unit 113 may extract the item from text data by searching the words recorded in the database.

Further, the extraction unit 113 may extract the item, for example, by using artificial intelligence (AI) trained using text data as input data so as to output the item.

When the extraction unit 113 extracts numerical data, it may convert the degree of the item into numbers by extracting a value indicating the degree of the item described in text data.

For example, when "the reaction solution was heated at a reaction temperature of 60 degrees centigrade for one hour" is described in text data, the extraction unit 113 first extracts the "reaction temperature" and the "reaction time" as the items related to the experimental conditions. Then, for the item of the "reaction temperature", the extraction unit 113 convers the degree of the item into numbers by extracting the numerical value of "60 degrees centigrade". Further, for the item of the "reaction time", the extraction unit 113 converts the degree of the item into numbers by extracting the numerical value of "one hour".

Further, when the extraction unit 113 extracts the item related to the physical properties of samples, the extraction unit 113 may convert the degrees of the physical properties into numbers by assigning a first predetermined value to the sample that exhibits the physical property and a second predetermined value to the sample that does not exhibit the physical property.

For example, when text data in which "a product having a foaming property was obtained" is described and text data in which "a product was obtained" is described without mentioning the foaming property of the product are acquired, the extraction unit 113 first extracts the "foaming property" as the item related to the physical properties of the samples.

Then the extraction unit 113 assigns a first predetermined value (e.g., "1") to the item of the "foaming property" of the sample corresponding to the text data in which "a product having a foaming property was obtained" is described. Further, the extraction unit 113 assigns a second predetermined value (for example, "0") to the item of the "foaming property" of the sample corresponding to the text data in which "a product was obtained" is described without mentioning the foaming property of the product.

However, when the extraction unit 113 extracts the item related to the physical properties of the samples, the extraction unit 113 may simply extract identification information about whether or not the physical properties are exhibited without converting the degrees of the physical properties into numbers.

Further, the extraction unit 113 may extract a word indicating the degree of the item described in text data, and convert the degree of the item based on the extracted word into numbers.

For example, when there are text data in which "a slightly-foaming product was obtained" is described, text data in which "a foaming product was obtained" is described, and text data in which "a vigorously-foaming product was obtained" is described, the extraction unit 113 first extracts the "foaming property" as an item related to the physical properties of samples. Further, the extraction unit 113 extracts "slightly" and "vigorously" as words indicating the degree of the item.

Then the extraction unit 113 assigns a first predetermined value (e.g., "1") to the item of the "foaming property" of the sample corresponding to the text data in which "a slightly-foaming product was obtained" is described. Further, the extraction unit 113 assigns a second predetermined value (e.g., "2") larger than the first predetermined value to the item of the "foaming property" of the sample corresponding to the text data in which "a foaming product was obtained" is described. Furthermore, the extraction unit 113 assigns a third predetermined value (e.g., "3") larger than the second predetermined value to the item of the "foaming property" of the sample corresponding to the text data in which "a vigorously-foaming product was obtained" is described.

Further, the extraction unit 113 may extract identification information about each of the extracted items as extraction information.

In this case, the extraction unit 113 may, for example, extract the "production lot number of a compound" as an item and extract the corresponding "production lot number" as identification information.

Further, the extraction unit 113 may, for example, extract the "date when a sample was prepared" as an item and extract the corresponding "date" as identification information.

Further, the extraction unit 113 may extract, for example, the "sample preparation method" as an item. In this case, the extraction unit 113 may assign an identifier to the type of the "sample preparation method" in advance. Then the extraction unit 113 may identify the corresponding sample preparation method based on information recorded in text data, and record an identifier corresponding to the identified preparation method as extraction information.

As described above, the extraction unit 113 according to this embodiment extracts extraction information about experiment contents from text data. However, a method for extracting it is not limited to a particular method. That is, the extraction unit 113 according to this embodiment may extract extraction information using any method by which it is possible to extract, from text data, information with which samples can be classified and evaluated.

Note that the extraction unit 113 according to this embodiment may extract extraction information from text data by using, for example, artificial intelligence (AI).

In this case, the extraction unit 113 extracts extraction information by using artificial intelligence (AI) trained using the text data as input data so as to output extraction data in a form with which samples can be classified and evaluated in the display control unit 114 described later.

The display control unit 114 acquires extraction information from the extraction unit 113. The display control unit 114 causes a display apparatus of the user terminal 200 to display at least one of analysis subject data and a result of analysis of the analysis subject data. More specifically, the display control unit 114 displays at least one of analysis subject data and a result of analysis of the analysis subject data in a differentiated manner in accordance with a difference between pieces of the extraction information.

Note that when the display control unit 114 displays a result of analysis of analysis subject data, it may acquire the result of analysis from an analysis unit not shown in Fig. 3. In this case, the analysis unit acquires the analysis subject data from the analysis subject data acquisition unit 111, performs analysis on the acquired analysis subject data, and outputs a result of the analysis.

The display control unit 114 may, for example, group samples into a plurality of groups in accordance with a difference between pieces of extraction information. Then the display control unit 114 may display at least one of analysis subject data and a result of analysis of the analysis subject data in different forms for each of the groups.

Fig. 4 is a schematic screen diagram showing the configuration of the server according to the first embodiment. More specifically, Fig. 4 is a diagram showing a specific example of a screen to be displayed on the user terminal 200 by the display control unit 114 according to this embodiment.

In Fig. 4, the display control unit 114 groups samples into a group of samples "having foaming properties" and a group of samples "having no foaming properties".

Then the display control unit 114 displays spectrum data D1 of the samples belonging to the group of samples "having foaming properties" as a solid line and spectrum data D2 of the samples belonging to the group of samples "having no foaming properties" as a broken line.

That is, in Fig. 4, the display control unit 114 groups samples into a plurality of groups in accordance with a difference in the item of the "foaming property" between the samples, and displays spectrum data, which is analysis subject data, using different lines for each group.

According to the above configuration, a user can visually and intuitively know a difference between pieces of analysis subject data corresponding to a difference between pieces of extraction information.

Note that, in Fig. 4, display forms of spectrum data are differentiated by using solid and broken lines. However, a method for differentiating display forms of spectrum data is not limited thereto. For example, the display control unit 114 according to the present disclosure may differentiate display forms of spectrum data by using lines of different colors.

In other words, when analysis subject data includes measurement data, the display control unit 114 according to the present disclosure may display the measurement data in different colors for each group.

Further, in Fig. 4, only two pieces of spectrum data are displayed. However, the number of pieces of spectrum data that can be displayed is not limited to two, and may instead be, for example, three or larger.

Further, in Fig. 4, the display control unit 114 displays spectrum data. However, analysis subject data that can be displayed in the above-described form is not limited to spectrum data. For example, the display control unit 114 may display waveform data or the like in the above-described form.

Further, in Fig. 4, the display control unit 114 displays analysis subject data. However, the display control unit 114 may display a result of analysis of analysis subject data in the above-described form. Examples of a result of analysis of analysis subject data that can be displayed in the above-described form include spectrum data and feature data extracted from waveform data or the like.

As described above, in Fig. 4, the display control unit 114 groups samples into a plurality of groups, and displays spectrum data, which is analysis subject data, using different lines for each group.

However, the display control unit 114 according to the present disclosure may display at least one of analysis subject data and a result of analysis of the analysis subject data without grouping samples into a plurality of groups.

In this case, the display control unit 114 may acquire, for example, numerical data obtained by converting the degree of an item into numbers from the extraction unit 113. Then the display control unit 114 may display at least one of the analysis subject data and the result of analysis of the analysis subject data by using lines whose color densities change in accordance with the acquired numerical data. Specifically, for example, the display control unit 114 may display the corresponding spectrum data by using a line of a darker color as the sample takes a longer reaction time.

According to the above configuration as well, the display control unit 114 can display at least one of analysis subject data and a result of analysis of the analysis subject data in a differentiated manner in accordance with a difference between pieces of extraction information.

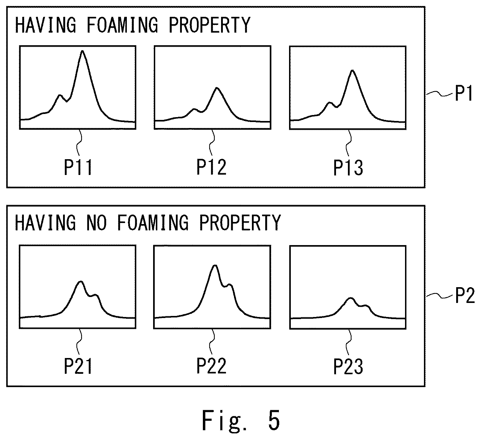

Fig. 5 is a schematic screen diagram showing the configuration of the server according to the first embodiment. More specifically, Fig. 5 is a diagram showing a specific example of a screen to be displayed on the user terminal 200 by the display control unit 114 according to this embodiment.

In Fig. 5, the display control unit 114 groups samples into a group of samples "having foaming properties" and a group of samples "having no foaming properties".

Then the display control unit 114 displays images P11, P12, and P13 showing analysis subject data of the samples belonging to the group of samples "having foaming properties" in a first display area P1, and displays images P21, P22, and P23 showing analysis subject data of the samples belonging to the group of samples "having no foaming properties" in a second display area P2.

That is, in Fig. 5, the display control unit 114 groups samples into a plurality of groups in accordance with a difference in the item of the "foaming property" between the samples, and displays images showing analysis subject data in different display areas for each of the groups.

According to the above configuration, a user can visually and intuitively know a difference between pieces of analysis subject data corresponding to a difference between pieces of extraction information.

Note that, in Fig. 5, three images are displayed in each area. However, the number of images that can be displayed is not limited to three, and may instead be one, two, or four or larger. Further, an area where no image is displayed may be present.

The display control unit 114 may display images showing, for example, spectrum data or waveform data on the screen shown in Fig. 5. Further, the display control unit 114 may display analysis subject data acquired as two-dimensional image data or three-dimensional image data on the screen shown in Fig. 5.

Further, in Fig. 5, the display control unit 114 displays images showing analysis subject data. However, the display control unit 114 may display images showing results of analysis of the analysis subject data in the above-described form. Examples of images showing results of analysis of the analysis subject data which can be displayed in the above-described form include images showing features extracted from spectrum data, waveform data, two-dimensional image data, and three-dimensional image data.

### (Operation of Display system)

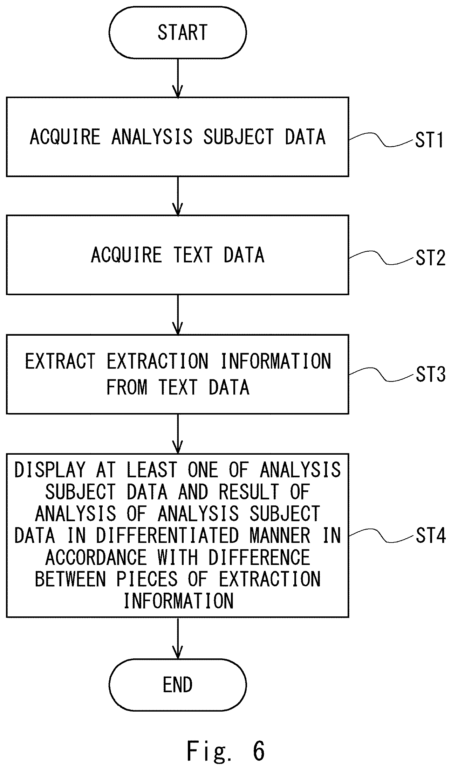

Next, operations performed by the display system, that is, a display method according to the first embodiment, will be described in detail. Fig. 6 is a flowchart showing operations performed by the display system according to the first embodiment. Note that, in the following description, Figs. 1 to 5 will be referred to as appropriate.

In the processing procedure shown in Fig. 6, the processor 110 of the server 100 functions as the analysis subject data acquisition unit 111, the text acquisition unit 112, the extraction unit 113, and the display control unit 114 by loading a program(s) stored in the storage device 130 into the memory 120 and executing the loaded program(s).

In the display method according to this embodiment, first, the processor 110 acquires analysis subject data (Step ST1). More specifically, in Step ST1, the processor 110 acquires analysis subject data which is at least one of measurement data obtained by measuring samples and numerical data about the samples. That is, in Step ST1, the processor 110 functions as the analysis subject data acquisition unit 111.

For example, in Step ST1, the processor 110 receives selection of analysis subject data to be analyzed from the user terminal 200. Then the processor 110 acquires the selected analysis subject data from the storage device 130.

Next, the processor 110 acquires text data (Step ST2). More specifically, in Step ST2, the processor 110 acquires text data about the samples. That is, in Step ST2, the processor 110 functions as the text acquisition unit 112.

For example, in Step ST2, the processor 110 receives selection of text data from the user terminal 200. Then the processor 110 may acquire the selected text data from the storage device 130. Further, in Step ST2, the processor 110 may acquire the text data, which is associated with the analysis subject data acquired in Step ST1 and stored, from the storage device 130.

Note that the order in which Steps ST1 and ST2 are performed may be reversed. Further, Steps ST1 and ST2 may be performed in parallel.

Next, the processor 110 extracts extraction information from the text data (Step ST3). More specifically, in Step ST3, the processor 110 extracts extraction information about experiment contents from the text data. That is, in Step ST3, the processor 110 functions as the extraction unit 113.

Note that, as described above, extraction information according to this embodiment may include, for example, at least one of information about the experimental conditions of samples and information about the physical properties of the samples.

Lastly, the processor 110 displays at least one of the analysis subject data and a result of analysis of the analysis subject data in a differentiated manner in accordance with a difference between pieces of the extraction information (Step ST4), and the display system 1 ends the series of operations. That is, in Step ST4, the processor 110 functions as the display control unit 114.

In Step ST4, the processor 110 causes the display apparatus of the user terminal 200 to display, for example, screens as shown in Figs. 4 and 5.

As described above, the display system 1 according to this embodiment extracts extraction information from text data, and displays at least one of analysis subject data and a result of analysis of the analysis subject data in a differentiated manner in accordance with a difference between pieces of the extraction information.

According to the above configuration, a user can intuitively know how the extracted difference between samples is reflected in analysis subject data and a result of analysis of the analysis subject data. As a result, the display system 1 according to this embodiment can assist analysis that utilizes knowledge possessed by an experimenter.

<Second Embodiment>

### (Configuration of Display system)

A second embodiment according to the present disclosure will be described hereinafter in detail with reference to the drawings. A display system according to this embodiment is an application example of the display system according to the first embodiment.

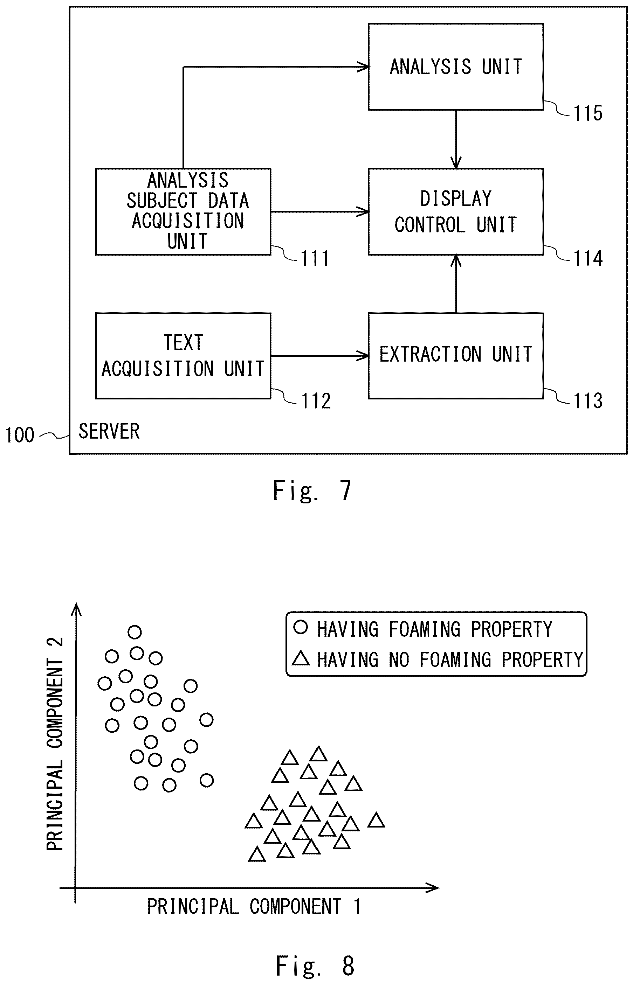

Fig. 7 is a block diagram showing a configuration of a server according to the second embodiment. The display system according to this embodiment differs from that according to the first embodiment in that the server 100 includes an analysis unit 115, and configurations other than the above one are similar to those of the display system according to the first embodiment.

The analysis unit 115 according to this embodiment acquires analysis subject data from the analysis subject data acquisition unit 111. Then the analysis unit 115 plots plot points corresponding to samples on mapping data based on the analysis subject data.

For example, the analysis unit 115 performs principal component analysis on the acquired analysis subject data, and acquires principal components and principal component scores as a result of the principal component analysis.

Then the analysis unit 115 may create mapping data using the acquired principal components and values indicating the performance or the physical properties of the samples corresponding to the analysis subject data as axes, and plot plot points corresponding to the samples on the created mapping data.

Further, the analysis unit 115 may create mapping data using the acquired principal components of two or more types as axes, and plot plot points corresponding to the samples on the created mapping data.

Note that mapping data output by the analysis unit 115 is not limited to mapping data indicating a result of principal component analysis. The analysis unit 115 may output any mapping data which can be used to analyze samples.

Note that the mapping data described herein is included in a result of analysis of analysis subject data.

Fig. 8 is a schematic screen diagram showing the configuration of the server according to the second embodiment. More specifically, Fig. 8 is a diagram showing a specific example of a screen to be displayed on the user terminal 200 by the display control unit 114 according to this embodiment.

In Fig. 8, the display control unit 114 groups samples into a group of samples "having foaming properties" and a group of samples "having no foaming properties".

Then the display control unit 114 displays plot points corresponding to the samples belonging to the group of samples "having foaming properties" by circles and plot points corresponding to the samples belonging to the group of samples "having no foaming properties" by triangles.

That is, in Fig. 8, the display control unit 114 groups samples into a plurality of groups in accordance with a difference in the item of the "foaming property" between the samples, and displays plot points in different forms for each of the groups.

According to the above configuration, a user can visually and intuitively know a difference in distribution between samples corresponding to a difference between pieces of extraction information.

### <Other Embodiments>

Although the display system according to each of the first and the second embodiments is implemented as the server 100, the configuration of the display system according to the present disclosure is not limited thereto. For example, the display system according to the present disclosure may be implemented by two or more computer apparatuses. Further, for example, some or all of the configurations of the display system according to the present disclosure may be implemented as those of the user terminal 200.

Although the present disclosure has been described above with reference to the above embodiments, the present disclosure is not limited only to the configurations of the above-described embodiments. Needless to say, the present disclosure includes various modifications, changes, and combinations that can be made by a person skilled in the art within the scope of the disclosure set forth in the claims of the present application.

From the disclosure thus described, it will be obvious that the embodiments of the disclosure may be varied in many ways. Such variations are not to be regarded as a departure from the spirit and scope of the disclosure, and all such modifications as would be obvious to one skilled in the art are intended for inclusion within the scope of the following claims.

## Claims

1. A display system comprising:
an analysis subject data acquisition unit (111) configured to acquire analysis subject data including at least one of measurement data obtained by measuring samples and numerical data about the samples;
a text acquisition unit (112) configured to acquire text data about the samples;
an extraction unit (113) configured to extract extraction information about the samples from the text data; and
a display control unit (114) configured to display at least one of the analysis subject data and a result of analysis of the analysis subject data in a differentiated manner in accordance with a difference between pieces of the extraction information.

2. The display system according to claim 1, wherein the extraction information includes at least one of information about experimental conditions of the samples and information about physical properties of the samples.

3. The display system according to claim 1 or 2, wherein the display control unit (114) groups the samples into a plurality of groups in accordance with the difference between pieces of the extraction information, and displays at least one of the analysis subject data and the result of analysis of the analysis subject data in different forms for each of the groups.

4. The display system according to claim 3, wherein
the analysis subject data includes the measurement data, and
the display control unit (114) displays the measurement data in different colors for each of the groups.

5. The display system according to claim 3, wherein the display control unit (114) displays at least one of the analysis subject data and the result of analysis of the analysis subject data in different display areas for each of the groups.

6. The display system according to claim 3, further comprising an analysis unit (115) configured to plot plot points corresponding to the samples on mapping data based on the analysis subject data,
wherein the display control unit (114) displays the plot points in different forms for each of the groups.

7. A display method comprising:
acquiring analysis subject data including at least one of measurement data obtained by measuring samples and numerical data about the samples;
acquiring text data about the samples;
extracting extraction information about the samples from the text data; and
displaying at least one of the analysis subject data and a result of analysis of the analysis subject data in a differentiated manner in accordance with a difference between pieces of the extraction information.

8. A display program for causing a computer to perform operations including:
acquiring analysis subject data including at least one of measurement data obtained by measuring samples and numerical data about the samples;
acquiring text data about the samples;
extracting extraction information about the samples from the text data; and
displaying at least one of the analysis subject data and a result of analysis of the analysis subject data in a differentiated manner in accordance with a difference between pieces of the extraction information.
